# FASCICULE DE BREVET EUROPEEN

(11) **EP 1 584 326 B1**
(45) Date de publication et mention de la délivrance du brevet: **23.11.2016**
(21) Numéro de dépôt: 05300246.5
(22) Date de dépôt: 01.04.2005
(51) Int. Cl.: A61K 8/84, A61Q 5/04

(54) **Procédé de traitment capillaire et utilisation dudit procédé**
Haarbehandlungsverfahren und dessen Einsatz
Hair treatment method and its use

(30) Priorité: 02.04.2004 FR 0450669
(43) Date de publication de la demande: 12.10.2005
(73) Titulaire: L'Oréal, 75008 Paris (FR)
(72) Inventeur: Fondin, Thomas, 95150 Taverny (FR); Sabbagh, Anne, 92500 Rueil Malmaison (FR)
(74) Mandataire: Caillet, Isabelle

(56) Documents cités:
- FR-A- 1 085 921
- FR-A- 2 514 640
- US-A- 3 472 243
- US-A- 5 279 818
- US-A- 6 013 249
- DATABASE WPI Section Ch, Week 200107 Derwent Publications Ltd., London, GB; Class D21, AN 2001-052903 XP002307806 & JP 2000 256146 A (MIRUBON KK) 19 septembre 2000 (2000-09-19) -& JP 2000 256146 A 19 septembre 2000 (2000-09-19)
- DATABASE WPI Section Ch, Week 200170 Derwent Publications Ltd., London, GB; Class D21, AN 2001-609747 XP002307805 -& JP 2001 213741 A (HOYU KK) 7 août 2001 (2001-08-07)

## Description

La présente invention se rapporte à un procédé de traitement des fibres capillaires, ainsi qu'à l'utilisation dudit procédé.

Il est usuel, pour obtenir la déformation permanente des cheveux, de réaliser dans un premier temps l'ouverture des liaisons disulfures de la kératine (cystine) à l'aide d'une composition contenant un agent réducteur, puis après avoir de préférence rincé les cheveux, à reconstituer dans un second temps lesdites liaisons disulfures en appliquant sur les cheveux lissés ou préalablement mis sous tension par des moyens adéquats tels que des bigoudis ou autres ou mis en forme ou lissés par d'autres moyens, une composition oxydante encore appelée fixateur de façon à donner à la chevelure la forme recherchée. Cette technique permet indifféremment de réaliser soit l'ondulation des cheveux, soit leur défrisage, leur crêpage ou leur lissage.

Les compositions réductrices utilisables pour la mise en oeuvre de la première étape de ces procédés contiennent généralement des composés thiolés, tels que l'acide thioglycolique, la cystéine, la cystéamine, l'acide thiolactique et le monothioglycolate de glycérol.

Une telle technique ne donne pas entièrement satisfaction. En effet, cette technique est très efficace pour modifier la forme des cheveux, mais aussi très dégradante pour les fibres capillaires.

Il a en outre été proposé d'élever la température des cheveux, entre l'étape de réduction et l'étape de fixation, au moyen d'un fer chauffant.

Ainsi, la demande de brevet JP 2000 256146 décrit un procédé de déformation permanente des cheveux comprenant l'application d'une composition cosmétique contenant 2 à 11% d'agents réducteurs et de 0,2 à 4% de dithiodiglycolate de diammonium. L'application de la composition réductrice est suivie du passage d'un fer chauffant entre 60 et 220°C.

Cependant, une telle technique d'utilisation d'un fer chauffant nécessite en outre une étape de fixation après le passage du fer, ce qui augmente la durée du traitement.

De plus, la forme obtenue est irréversible. Le contraste entre les parties traitées et les racines est alors important au moment de la repousse des cheveux.

Enfin, si le traitement est effectué sur des cheveux colorés, on constate très souvent une altération de la couleur due au traitement.

Le but de la présente invention est donc de fournir un procédé de traitement des fibres capillaires qui remédie aux inconvénients de l'art antérieur.

En particulier, la présente invention a pour but de fournir un procédé de traitement des fibres capillaires qui permet de modifier le comportement de la fibre capillaire en limitant son altération, de maîtriser le volume des cheveux et d'améliorer les performances cosmétiques des cheveux, notamment la douceur, la brillance et le démêlage en respectant mieux la couleur des cheveux teints.

Ledit procédé doit permettre en outre aux cheveux de garder un aspect naturel, ce qui limite l'effet racines, c'est-à-dire le contraste entre les parties traitées et les racines.

Enfin, l'invention a pour but de réduire la durée du traitement des fibres capillaires et d'obtenir des effets durables.

La Demanderesse a trouvé qu'il était possible de remédier aux inconvénients de l'art antérieur et de remplir les objectifs précités, en mettant en oeuvre un procédé de traitement des fibres capillaires sans fixation comprenant une étape d'application sur les fibres capillaires d'une composition réductrice sans céramide contenant au moins un agent réducteur thiolé et au moins un actif cosmétique, et le ou les actifs cosmétiques étant choisis parmi les actifs polymériques, puis une étape d'élévation de la température des fibres capillaires, au moyen d'un fer chauffant, à une température au moins égale à 60°C, l'élévation de la température étant effectuée avant ou après le rinçage éventuel des fibres capillaires.

Ainsi, l'invention a pour objet un procédé de traitement des fibres capillaires sans fixation comprenant les étapes suivantes :
- application sur les fibres capillaires d'une composition réductrice sans céramide contenant au moins un agent réducteur et au moins un actif cosmétique, le ou les agents réducteurs étant choisis parmi les thiols, et le ou les actifs cosmétiques étant choisis parmi les actifs polymériques,
- élévation de la température des fibres capillaires, au moyen d'un fer chauffant, à une température au moins égale à 60°C, l'élévation de la température étant effectuée avant ou après le rinçage éventuel des fibres capillaires.

Sans fixation signifie au sens de la présente invention sans application supplémentaire d'une composition contenant un oxydant chimique tel que par exemple le peroxyde d'hydrogène ou un bromate.

De préférence la composition réductrice ne contient pas d'acide dithiodiglycolique ou l'un de ses sels.

Le ou les actifs cosmétiques polymériques sont généralement choisis parmi les silicones volatiles ou non, linéaires ou cycliques, les polymères non siliconés cationiques, anioniques ou amphotères.

Comme silicones utilisables comme actifs cosmétiques dans le procédé selon l'invention, on peut citer les polydiméthylsiloxanes, les polyorganosiloxanes quaternisés tels que ceux décrits dans la demande de brevet français FR 2 535 730, les polyorganosiloxanes à groupements aminoalkyles modifiés par des groupements alcoxycarbonylalkyles tels que ceux décrits dans le brevet US n° 4 749 732, des polyorganosiloxanes tels que le copolymère polydiméthylsiloxane- polyoxyalkyle du type diméthicone copolyol, un polydiméthylsiloxane à groupements terminaux stéaroxy (stéaroxydiméthicone), un copolymère polydiméthylsiloxane-dialkylammonium acétate ou un copolymère polydiméthyl-siloxane polyalkylbétaïne décrits dans le brevet britannique GB 2 197 352, des polysiloxanes organo modifiés par des groupements mercapto ou mercaptoalkyles tels que ceux décrits dans le brevet français FR 1 530 369 et dans la demande de brevet européen EP 295 780.

Comme expliqué précédemment, le ou les actifs polymériques peuvent être aussi choisis parmi les polymères cationiques non siliconés.

Par "polymère cationique", on entend tout polymère contenant des groupements cationiques et/ou des groupements ionisables en groupements cationiques.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont notamment décrits dans les brevets français FR 2 505 348 et FR 2 542 997. Parmi ces polymères, on peut citer :
(1) les homopolymères ou copolymères dérivés d'esters ou d'amides de l'acide acrylique ou méthacrylique ;
(2) les dérivés d'éthers de cellulose comportant des groupements ammonium quaternaire décrits dans le brevet français FR 1 492 597 ;
(3) les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl-ou hydroxypropyl-celluloses greffées notamment avec un sel de méthacryloyléthyl-triméthylammonium, de méthacrylamidopropyl-triméthylammonium ou de diméthyldiallylammonium ; on peut citer par exemple le polyquaternium 10 (nom INCI) ;
(4) les autres polysaccharides cationiques décrits plus particulièrement dans les brevets US 3 589 578 et US 4 031 307 tels que les gommes de guar contenant des groupements cationiques trialkylammonium ;
(5) les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français FR 2 162 025 et FR 2 280 361 ;
(6) les polyaminoamides solubles dans l'eau, tels que ceux notamment décrits dans les brevets français FR 2 252 840 et FR 2 368 508 ;
(7) les dérivés de polyaminoamides, par exemple, les polymères acide adipique/dialkylaminohydroxyalkyldialkylène-triamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français FR 1 583 363 ;
(8) les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polyamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains US 3 227 615 et US 2 961 347;
(9) les cyclopolymères d'alkyldiallylamine ou de dialkyldiallylammonium tels que l'homopolymère de chlorure de diméthyldiallyl-ammonium et les copolymères de chlorure de diallyldiméthylammonium et d'acrylamide ;
(10) les polymères de diammonium quaternaire présentant une masse moléculaire moyenne en nombre généralement comprise entre 1000 et 100000, tels que, ceux décrits, par exemple, dans les brevets français FR 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020 ; on peut citer par exemple le chlorure d'hexadiméthrine (nom INCI), commercialisé par la société CHIMEX sous la référence MEXOMERE PO ;
(11) les polymères de polyammonium quaternaire tels que ceux notamment décrits dans la demande de brevet EP-A-122 324 ;
(12) les polymères quaternaires de vinylpyrrolidone et de vinylimidazole tels que, par exemple, les produits commercialisés sous les dénominations Luviquat^{®} FC 905, FC 550 et FC 370 par la société B.A.S.F;
(13) les polyamines comme le Polyquart^{®} H vendu par HENKEL, référencées sous le nom de " POLYETHYLENEGLYCOL (15) TALLOW POLYAMINE " dans le dictionnaire CTFA ;
(14) les polymères réticulés de sels de méthacryloyloxyalkyl(C₁-C₄) trialkyl(C₁-C₄)ammonium tels que ceux commercialisés sous le nom de SALCARE^{®} SC 92, SALCARE^{®} SC 95 et SALCARE^{®} SC 96 par la Société ALLIED COLLOIDS ; et leurs mélanges.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

De préférence, les polymères cationiques sont choisis parmi le chlorure d'hexadiméthrine et les homo ou copolymères de chlorure de diméthyldiallylammonium.

Le ou les actifs polymériques peuvent encore être choisis parmi les polymères amphotères.

Les polymères amphotères utilisables conformément à l'invention peuvent être choisis parmi les polymères comportant des motifs B et C répartis statistiquement dans une chaîne polymère, où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes ; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus que l'on préfère plus particulièrement, sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylates et acrylates, les dialkylaminoalkyl-méthacrylamides et acrylamides. De tels composés sont décrits dans le brevet américain n° 3 836 537. On peut également citer le copolymère acrylate de sodium/chlorure d'acrylamidopropyltriméthyl-ammonium vendu sous la dénomination POLYQUART^{®} KE 3033 par la Société HENKEL.
   Le composé vinylique peut être également un sel de dialkyldiallylammonium tel que le chlorure de diéthyldiallyl-ammonium.
   Les copolymères d'acide acrylique et de ce dernier monomère sont commercialisés sous les appellations MERQUAT^{®} 280, MERQUAT^{®} 295 et MERQUAT^{®} PLUS 3330 par la société CALGON.
(2) Les polymères comportant des motifs dérivant :
   a) d'au moins un monomère choisi parmi les acrylamides ou les méthacrylamides substitués sur l'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.

   Les acrylamides ou méthacrylamides N-substitués plus particulièrement préférés selon l'invention sont les groupements dont les groupes alkyle contiennent de 2 à 12 atomes de carbone et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed, 1991) est Octylacrylamide/acrylates/butylaminoethyl-methacrylate copolymer tels que les produits vendus sous la dénomination AMPHOMER^{®} ou LOVOCRYL^{®} 47 par la société NATIONAL STARCH.
(3) Les polyaminoamides réticulés et alkylés partiellement ou totalement dérivant de polyaminoamides de formule générale : 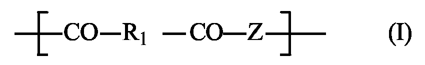 dans laquelle R₁ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis primaire ou bis dérivé secondaire, et Z désigne un groupe d'une polyalkylène-polyamine bis-primaire, mono ou bis-secondaire et représente de préférence :
   a) dans les proportions de 60 à 100 % en moles, le groupe 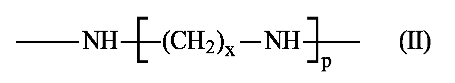 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (II) ci-dessus, dans lequel x=2 et p=1 et qui dérive de l'éthylènediamine, ou le groupe dérivant de la pipérazine 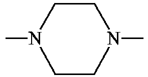
   c) dans les proportions de 0 à 20 % en moles, le groupe -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane-sultone ou de leurs sels.

   Les acides carboxyliques ou dicarboxyliques utilisables pour R₁ sont choisis de préférence parmi les acides ayant 6 à 10 atomes de carbone tels que les acides adipique, 2,2,4-triméthyladipique et 2,4,4-triméthyladipique, téréphtalique, les acides à double liaison éthylénique comme, par exemple, les acides acrylique, méthacrylique, itaconique.
   Les alcane-sultones utilisées dans l'alcoylation sont de préférence la propane- ou la butane-sultone. Les sels des agents d'alcoylation sont de préférence les sels de sodium ou de potassium.
(4) Les polymères comportant des motifs zwittérioniques de formule : 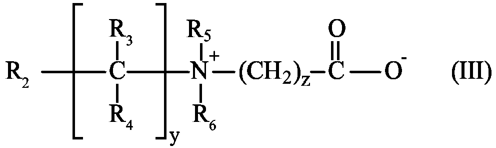 dans laquelle R₂ désigne un groupement insaturé polymérisable tel qu'un groupement acrylate, méthacrylate, acrylamide ou méthacrylamide, y et z représentent chacun un nombre entier de 1 à 3, R₃ et R₄ représentent un atome d'hydrogène, un groupement méthyle, éthyle ou propyle, R₅ et R₆ représentent un atome d'hydrogène ou un groupe alkyle de telle façon que la somme des atomes de carbone dans R₅ et R₆ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle, ou des acrylates ou méthacrylates d'alkyle, des acrylamides ou méthacrylamides, ou l'acétate de vinyle.
   A titre d'exemple, on peut citer le copolymère de méthacrylate de méthyle et de diméthyl-carboxyméthylammonio-éthylméthacrylate de méthyle tel que le produit vendu sous la dénomination DIAFORMER^{®} Z301 par la société SANDOZ.
(5) Les polymères dérivés du chitosane comportant des motifs monomères répondant aux formules suivantes: 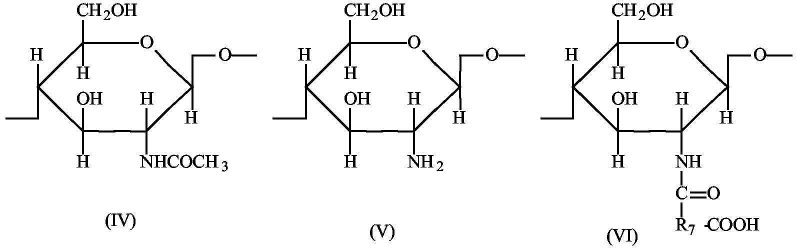 le motif (IV) étant présent dans des proportions comprises entre 0 et 30 %, le motif (V) dans des proportions comprises entre 5 et 50 % et le motif (VI) dans des proportions comprises entre 30 et 90 %, étant entendu que dans ce motif (VI), R₇ représente un groupe de formule: 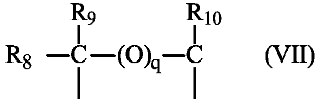 dans laquelle si q=0, R₈, R₉ et R₁₀, identiques ou différents, représentent chacun un atome d'hydrogène, un reste méthyle, hydroxyle, acétoxy ou amino, un reste monoalkylamine ou un reste dialkylamine éventuellement interrompus par un ou plusieurs atomes d'azote et/ou éventuellement substitués par un ou plusieurs groupes amine, hydroxyle, carboxyle, alkylthio, sulfonique, un reste alkylthio dont le groupe alkyle porte un reste amino, l'un au moins des groupes R₈, R₉ et R₁₀ étant dans ce cas un atome d'hydrogène ;
   ou si q=1, R₈, R₉ et R₁₀ représentent chacun un atome d'hydrogène, ainsi que les sels formés par ces composés avec des bases ou des acides.
(6) Les polymères dérivés de la N-carboxyalkylation du chitosane comme le N-carboxyméthyl-chitosane ou le N-carboxybutyl-chitosane vendu sous la dénomination EVALSAN^{®} par la société JAN DEKKER.
(7) Les polymères répondant à la formule générale (VIII) décrits par exemple, dans le brevet français FR 1 400 366: 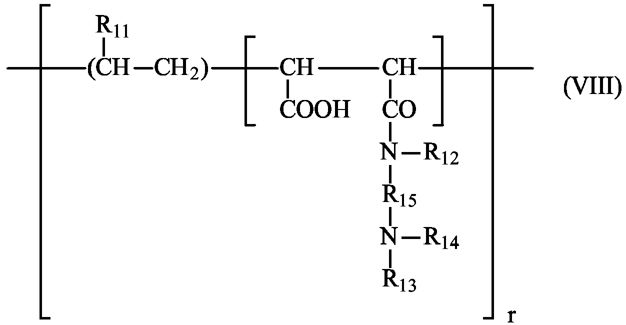 dans laquelle R₁₁ représente un atome d'hydrogène, un groupe CH₃O, CH₃CH₂O, phényle, R₁₂ désigne l'hydrogène ou un groupe alkyle inférieur tel que méthyle, éthyle, R₁₃ désigne l'hydrogène ou un groupe alkyle inférieur tel que méthyle, éthyle, R₁₄ désigne un groupe alkyle inférieur tel que méthyle, éthyle ou un groupe répondant à la formule: -R₁₅-N(R₁₃)₂, R₁₅ représentant un groupement -CH₂-CH₂-, -CH₂-CH₂-CH₂-, -CH₂-CH(CH₃)-, R₁₃ ayant les significations mentionnées ci-dessus,
   ainsi que les homologues supérieurs de ces groupes et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule:

      -D-X-D-X-D- (IX)

      où D désigne un groupe 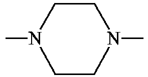 et X désigne le symbole E ou E', E ou E', identiques ou différents, désignent un groupe bivalent qui est un groupe alkylène à chaîne droite ou ramifiée comportant jusqu'à 7 atomes de carbone dans la chaîne principale non substituée ou substituée par des groupements hydroxyle, et pouvant comporter en outre des atomes d'oxygène, d'azote, de soufre, 1 à 3 cycles aromatiques et/ou hétérocycliques, les atomes d'oxygène, d'azote et de soufre étant présents sous forme de groupements éther, thioéther, sulfoxyde, sulfone, sulfonium, alkylamine, alcénylamine, hydroxyle, benzylamine, oxyde d'amine, ammonium quaternaire, amide, imide, alcool, ester et/ou uréthanne.
   b) Les polymères de formule :

      -D-X-D-X- (X)

      où D désigne un groupe 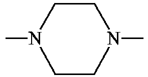 et X désigne le symbole E ou E" et au moins une fois E"; E ayant la signification indiquée ci-dessus et E" est un groupe bivalent qui est un groupe alkylène à chaîne droite ou ramifiée ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther/anhydride maléique modifié partiellement par semiamidification avec une N,N-dialkylaminoalkylamine telle que la N,N-diméthylaminopropyl-amine ou par semiestérification avec une N,N-dialcanolamine. Ces copolymères peuvent également comporter d'autres comonomères vinyliques tels que le vinylcaprolactame.

Le ou les actifs cosmétiques peuvent encore être choisis parmi les polymères anioniques.

Les polymères anioniques généralement utilisés dans la présente invention sont des polymères comportant des groupes dérivés d'acides carboxyliques, sulfoniques ou phosphoriques, et présentant une masse moléculaire en poids comprise entre 500 et 5 000 000.

Les groupements carboxyliques sont apportés par des monomères monoacides ou diacides carboxyliques insaturés tels que ceux répondant à la formule : 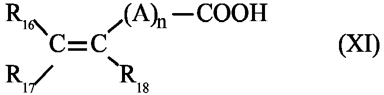 dans laquelle n est un nombre entier de 0 à 10, A désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₁₆ désigne un atome d'hydrogène, un groupement phényle ou benzyle, R₁₇ désigne un atome d'hydrogène, un groupement alkyle inférieur ou carboxyle, R₁₈ désigne un atome d'hydrogène, un groupement alkyle inférieur, un groupement -CH₂-COOH, phényle ou benzyle.

Dans la formule (XI) ci-dessus, le ou les groupements alkyles inférieurs comportent de préférence de 1 à 4 atomes de carbone et désignent, en particulier, les groupements méthyle et éthyle.

Les polymères anioniques à groupements carboxyliques préférés selon l'invention sont :
A) Les homo- ou copolymères d'acide acrylique ou méthacrylique ou leurs sels, et en particulier les produits vendus sous les dénominations VERSICOL^{®} E ou K par la société ALLIED COLLOID, ULTRAHOLD^{®} par la société BASF, les copolymères d'acide acrylique et d'acrylamide vendus sous la forme de leur sel de sodium sous les dénominations RETEN^{®} 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français FR 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER^{®} par la Société AMERICAN CYANAMID. On peut également citer les copolymères d'acide acrylique et de méthacrylate d'alkyle en C₁-C₄ et le copolymère d'acide méthacrylique et d'acrylate d'éthyle vendu sous la dénomination LUVIMER^{®} MAEX par la société BASF.
C) Les copolymères dérivés d'acide crotonique tels que ceux comportant dans leur chaîne des motifs acétate ou propionate de vinyle, et éventuellement d'autres monomères tels que les esters allyliques ou méthallyliques, éthers vinyliques ou esters vinyliques d'un acide carboxylique saturé, linéaire ou ramifié, à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique α ou β-cyclique. De tels polymères sont décrits entre autres dans les brevets français numéros FR 1 222 944, FR 1 580 545, FR 2 265 782, FR 2 265 781, FR 1 564 110 et FR 2 439 798. Des produits commerciaux entrant dans cette classe sont par exemple les résines 28-29-30, 26-13-14 et 28-13-10 vendues par la société NATIONAL STARCH.
D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters. Ces polymères peuvent être estérifiés. De tels polymères sont décrits en particulier dans les brevets US 2 047 398, US 2 723 248, US 2 102 113, et dans le brevet GB 839 805. On peut citer notamment ceux vendus sous les dénominations GANTREZ^{®} AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acryliques ou méthacryliques ou la vinylpyrrolidone dans leur chaîne, les fonctions anhydrides sont monoestérifiées ou monoamidifiées. Ces polymères sont par exemple décrits dans les brevets français FR 2 350 384 et FR 2 357 241 de la demanderesse.
E) Les polyacrylamides comportant des groupements carboxylates.

Comme expliqué précédemment, les polymères anioniques peuvent être également des polymères comportant des groupes dérivés d'acides sulfoniques.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000 ainsi que les copolymères avec un comonomère insaturé tel que les acides acrylique ou méthacrylique, et leurs esters, ainsi que l'acrylamide ou ses dérivés, les éthers vinyliques et la vinylpyrrolidone ;
- les sels de l'acide polystyrène -sulfonique, notamment les sels de sodium, ayant une masse moléculaire d'environ 500 000 et d'environ 100 000 vendus respectivement sous les dénominations Flexan^{®} 500 et Flexan^{®} 130 par National Starch. Ces composés sont décrits dans le brevet FR2198719;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER^{®} HSP 1180 par Henkel.

Les polymères de l'invention peuvent également être des polyuréthanes anioniques, cationiques, ou amphotères.

Les polymères de l'invention peuvent également être des polymères comportant au moins une chaîne grasse en C₁₀-C₃₀ dans leur structure (polymères fréquemment référencés sous le nom de polymères associatifs).

Le ou les actifs cosmétiques polymériques représentent généralement de 0,1 à 30 %, de préférence de 0,5 à 10 %, en poids par rapport au poids total de la composition réductrice.

Comme expliqué précédemment, la composition réductrice utilisée dans le procédé selon l'invention comprend un ou plusieurs agents réducteurs choisis parmi les thiols, éventuellement utilisés sous forme de sels.

De préférence, le ou les thiols utilisés comme agents réducteurs dans la composition réductrice sont choisis parmi la cystéïne et ses dérivés, comme la N-acétylcystéïne, la cystéamine et ses dérivés, comme ses dérivés acylés en C₁-C₄ tels que la N-acétyl cystéamine et la N-propionyl cystéamine, l'acide thiolactique et ses esters, comme le monothiolactate de glycérol, l'acide thioglycolique et ses esters, comme le monothioglycolate de glycérol, et le thioglycérol, ou leurs sels.

Comme thiols utilisables dans la composition réductrice utilisée selon l'invention, on peut encore citer les N-mercapto-alkylamides de sucres tels que le N-(mercapto-2-éthyl)gluconamide, l'acide β-mercaptopropionique et ses dérivés, l'acide thiomalique, la panthétéïne, les N-(mercaptoalkyl)ω-hydroxyalkylamides tels que ceux décrits dans la demande de brevet EP-A-354 835 et les N-mono- ou N,N-dialkylmercapto 4-butyramides tels que ceux décrits dans la demande de brevet EP-A-368 763, les aminomercaptoalkylamides tels que ceux décrits dans la demande de brevet EP-A-432 000 et les alkylaminomercaptoalkylamides tels que ceux décrits dans la demande de brevet EP-A-514 282, le mélange de thioglycolate d'hydroxy-2 propyle (2/3) et de thioglycolate d'hydroxy-2 méthyl-1 éthyle (67/33) décrit dans la demande de brevet FR-A-2 679 448.

Généralement, le ou les thiols utilisés dans la composition réductrice représentent de 0,1 à 30 %, de préférence de 0,5 à 20 %, mieux de 1 à 10 %, en poids par rapport au poids total de la composition réductrice.

Selon un mode de réalisation particulièrement préféré, le ou les thiols présents dans la composition réductrice représentent au total moins de 5% en poids du poids total de la composition réductrice.

Le pH de la composition réductrice est compris généralement entre 2 et 13, de préférence entre 6 et 10, mieux est inférieur ou égal à 9.

Le réglage du pH de la composition peut être obtenu à l'aide d'un agent alcalin, tel que, par exemple, l'ammoniaque ou une amine organique telle que le 2-amino-2-méthyl-1-propanol, la monoéthanolamine, la diéthanolamine, la triéthanolamine, la 1,3-propanediamine, un carbonate ou bicarbonate alcalin ou d'ammonium, un carbonate organique tel que le carbonate de guanidine, un hydroxyde alcalin, ou bien à l'aide d'un agent acidifiant tel que par exemple l'acide chlorhydrique, l'acide acétique, l'acide lactique, l'acide oxalique ou l'acide borique.

La composition réductrice comprend généralement un ou plusieurs solvants cosmétiquement acceptables, choisis de préférence parmi l'eau, les alcools en C₁-C₆, de préférence les alcanols tels que l'éthanol, le propanol et l'isopropanol, les polyols tels que le glycérol, le propylène glycol et le pentanediol, l'alcool benzylique, les éthers de polyols, les esters en C₂-C₆, la N-méthylpyrrolidone (NMP), et les cétones en C₃-C₆.

La composition réductrice utilisée dans le procédé selon l'invention peut se présenter sous la forme d'une lotion, épaissie ou non, d'une crème, d'un gel ou d'une mousse.

L'application de la composition réductrice telle que définie précédemment constitue donc la première étape du procédé selon l'invention.

De préférence, la composition réductrice est appliquée sur des fibres capillaires humides et propres.

Après l'application de la composition réductrice, on peut laisser poser ladite composition, généralement pendant 5 à 60 minutes, de préférence 5 à 30 minutes, éventuellement sous casque.

Comme expliqué précédemment, le procédé selon l'invention comprend, après l'étape d'application de la composition réductrice, une éventuelle étape de rinçage, puis une étape d'élévation de la température des fibres capillaires, au moyen d'un fer chauffant, à une température au moins égale à 60°C.

Au sens de la présente invention, on entend par fer un dispositif de chauffage des fibres capillaires par contact.

L'extrémité du fer venant en contact avec les cheveux peut avoir différentes formes. Elle peut notamment présenter une surface plane ; on parle dans ce cas de fer plat. Elle peut également présenter une surface arrondie ; on parle dans ce cas de fer rond.

L'application du fer peut se faire par touches séparées successives de quelques secondes, ou par déplacement ou glissement progressif le long des mèches.

A titre d'exemple de fer utilisable dans le procédé selon l'invention, on peut citer tous types de fer plats ou ronds et, en particulier, de manière non limitative, ceux décrits dans les brevets US 4 103 145, US 4 308 878, US 5 983 903, US 5 957 140, US 5 494 058 et US 5 046 516.

De préférence, la température des fibres capillaires est élevée jusqu'à une température comprise entre 60°C et 250°C, mieux entre 120°C et 220°C.

Selon un mode de réalisation préféré, les fibres capillaires ne sont pas rincées avant l'étape d'élévation de la température.

Le procédé selon l'invention peut également comprendre une étape supplémentaire de pré-séchage partiel des fibres capillaires avant l'étape d'élévation de la température, de manière à éviter d'importants dégagements de vapeurs qui pourraient brûler les mains du coiffeur et le cuir chevelu du sujet. Cette étape de pré-séchage peut se faire par exemple au moyen d'un séchoir, d'un casque, ou bien encore par séchage libre.

La présente invention a également pour objet l'utilisation du procédé tel que décrit précédemment pour modifier de manière durable la forme de la chevelure avec une faible dégradation de la couleur des cheveux et/ou avec une faible dégradation des fibres capillaires.

La présente invention est illustrée par les exemples suivant.

### Exemples

On met en oeuvre le procédé de traitement des fibres capillaires selon l'invention en utilisant une composition réductrice.

Les compositions réductrices testées sont les suivantes :

**Composition réductrice 1**

| | |
|---|---|
| L-Cystéine | 1,4 g |
| MEXOMERE PO | 2,5 g |
| 2-amino-2-méthyl-1-propanol | qs pH 9,5 |
| Eau déminéralisée | qsp 100 g |

**Composition réductrice 2**

| | |
|---|---|
| Acide thioglycolique | 1,1 g |
| MEXOMERE PO | 2,5 g |
| 2-amino-2-méthyl-1-propanol | qs pH 9,5 |
| Eau déminéralisée | qsp 100 g |

**Composition réductrice 3**

| | |
|---|---|
| L-Cystéine | 1,4 g |
| Acide thioglycolique | 0,3 g |
| MEXOMERE PO | 2,5 g |
| 2-amino-2-méthyl-1-propanol | qs pH 9,5 |
| Eau déminéralisée | qsp 100 g |

Les essais sont réalisés sur des cheveux colorés naturellement frisés.

La composition réductrice est appliquée sur la tête. On laisse poser la composition pendant 5 minutes.

On effectue ensuite un pré-séchage partiel des cheveux au moyen d'un sèche-cheveux.

Puis on passe un fer plat chauffé à 180°C

On constate en final un bon toucher de la fibre, une maîtrise du volume, un bon respect de la couleur et une bonne rémanence des effets dans le temps.

## Revendications

1. Procédé de traitement des fibres capillaires, sans application supplémentaire d'une composition contenant un oxydant chimique, **caractérisé en ce qu'**il comprend les étapes suivantes :
- application sur les fibres capillaires d'une composition réductrice sans céramide contenant au moins un agent réducteur et au moins un actif cosmétique, le ou les agents réducteurs étant choisis parmi les thiols, et le ou les actifs cosmétiques étant choisis parmi les actifs polymériques choisis parmi les silicones volatiles ou non, linéaires ou cycliques, les polymères non siliconés cationiques, anioniques ou amphotères,
- élévation de la température des fibres capillaires, au moyen d'un fer chauffant, à une température au moins égale à 60 °C, l'élévation de la température étant effectuée avant ou après le rinçage éventuel des fibres capillaires.

2. Procédé selon la revendication 1, **caractérisé en ce que** la composition réductrice ne contient pas d'acide dithiodiglycolique ou l'un de ses sels.

3. Procédé selon l'une quelconque des revendications précédentes, **caractérisé en ce que** le ou les actifs cosmétiques polymériques représentent de 0,1 à 30 %, de préférence de 0,5 à 10 %, en poids par rapport au poids total de la composition réductrice.

4. Procédé selon l'une quelconque des revendications précédentes, **caractérisé en ce que** le ou les thiols représentent de 0,1 à 30 %, de préférence de 0,5 à 20 %, mieux de 1 à 10 %, en poids par rapport au poids total de la composition réductrice.

5. Procédé selon la revendication 4, **caractérisé en ce que** le ou les thiols représentent moins de 5% en poids du poids total de la composition réductrice.

6. Procédé selon l'une quelconque des revendications précédentes, **caractérisé en ce que** le ou les thiols sont choisis parmi la cystéïne et ses dérivés, la cystéamine et ses dérivés, l'acide thiolactique et ses esters, l'acide thioglycolique et ses esters, le thioglycérol, ou leurs sels.

7. Procédé selon l'une quelconque des revendications précédentes, **caractérisé en ce que** la composition réductrice comprend un ou plusieurs solvants choisis parmi l'eau, les alcools en C₁-C₆, de préférence les alcanols tels que l'éthanol, le propanol et l'isopropanol, les polyols tels que le glycérol, le propylène glycol et le pentanediol, l'alcool benzylique, les éthers de polyols, les esters en C₂-C₆, la N-méthylpyrrolidone (NMP), et les cétones en C₃-C₆.

8. Procédé selon l'une quelconque des revendications précédentes, **caractérisé en ce que** la composition réductrice se présente sous la forme d'une lotion, épaissie ou non, d'une crème, d'un gel ou d'une mousse.

9. Procédé selon l'une quelconque des revendications précédentes, **caractérisé en ce que** la température est élevée jusqu'à une température comprise entre 60 °C et 250 °C, de préférence entre 120 °C et 220 °C.

10. Procédé selon l'une quelconque des revendications précédentes, **caractérisé en ce que** la composition réductrice est appliquée sur des fibres capillaires humides et propres.

11. Procédé selon l'une quelconque des revendications précédentes, **caractérisé en ce que**, après l'application de la composition réductrice, on laisse poser ladite composition réductrice avant l'application du fer.

12. Procédé selon l'une quelconque des revendications précédentes, **caractérisé en ce que** les fibres capillaires ne sont pas rincées avant l'étape d'élévation de la température.

13. Procédé selon l'une quelconque des revendications précédentes, **caractérisé en ce qu'**il comprend une étape de pré-séchage partiel des fibres capillaires avant l'étape d'élévation de la température.

14. Utilisation du procédé selon l'une quelconque des revendications précédentes, pour modifier de manière durable la forme de la chevelure avec une faible dégradation de la couleur des cheveux et/ou avec une faible dégradation des fibres.

## Patentansprüche

1. Verfahren zur Behandlung von Haarfasern ohne zusätzliche Anwendung einer Zusammensetzung, die ein chemisches Oxidationsmittel enthält, **dadurch gekennzeichnet, dass** es die folgenden Schritte umfasst:
- Aufbringen einer ceramidfreien reduzierenden Zusammensetzung, die mindestens ein Reduktionsmittel und mindestens einen kosmetischen Wirkstoff enthält, auf die Haarfasern, wobei das Reduktionsmittel bzw. die Reduktionsmittel aus Thiolen ausgewählt ist bzw. sind und der kosmetische Wirkstoff bzw. die kosmetischen Wirkstoffe aus polymeren Wirkstoffen, die aus linearen oder cyclischen, flüchtigen oder nichtflüchtigen Silikonen und kationischen, anionischen oder amphoteren Nichtsilikonpolymeren ausgewählt sind, ausgewählt ist bzw. sind,
- Erhöhen der Temperatur der Haarfasern mit Hilfe eines Heizeisens auf eine Temperatur von mindestens 60°C, wobei die Temperaturerhöhung vor oder nach fakultativem Spülen der Haarfasern durchgeführt wird.

2. Verfahren nach Anspruch 1, **dadurch gekennzeichnet, dass** die reduzierende Zusammensetzung keine Dithiodiglykolsäure oder ein Salz davon enthält.

3. Verfahren nach einem der vorhergehenden Ansprüche, **dadurch gekennzeichnet, dass** der polymere kosmetische Wirkstoff bzw. die polymeren kosmetischen Wirkstoffe 0,1 bis 30 Gew.-%, vorzugsweise 0,5 bis 10 Gew.-%, bezogen auf das Gesamtgewicht der reduzierenden Zusammensetzung, ausmacht bzw. ausmachen.

4. Verfahren nach einem der vorhergehenden Ansprüche, **dadurch gekennzeichnet, dass** das Thiol bzw. die Thiole 0,1 bis 30 Gew.-%, vorzugsweise 0,5 bis 20 Gew.-%, noch besser 1 bis 10 Gew.-%, bezogen auf das Gesamtgewicht der reduzierenden Zusammensetzung, ausmacht bzw. ausmachen.

5. Verfahren nach Anspruch 4, **dadurch gekennzeichnet, dass** das Thiol bzw. die Thiole weniger als 5 Gew.-% des Gesamtgewichts der reduzierenden Zusammensetzung ausmacht bzw. ausmachen.

6. Verfahren nach einem der vorhergehenden Ansprüche, **dadurch gekennzeichnet, dass** das Thiol bzw. die Thiole aus Cystein und Derivaten davon, Cysteamin und Derivaten davon, Thiomilchsäure und Estern davon, Thioglykolsäure und Estern davon, Thioglycerin oder Salzen davon ausgewählt ist bzw. sind.

7. Verfahren nach einem der vorhergehenden Ansprüche, **dadurch gekennzeichnet, dass** die reduzierende Zusammensetzung ein oder mehrere Lösungsmittel, die aus Wasser, C₁-C₆-Alkoholen, vorzugsweise Alkanolen wie Ethanol, Propanol und Isopropanol, Polyolen wie Glycerin, Propylenglykol und Pentandiol, Benzylalkohol, Polyolethern, C₂-C₆-Estern, N-Methylpyrrolidon (NMP) und C₃-C₆-Ketonen ausgewählt sind, umfasst.

8. Verfahren nach einem der vorhergehenden Ansprüche, **dadurch gekennzeichnet, dass** die reduzierende Zusammensetzung in Form einer verdickten oder unverdickten Lotion, einer Creme, eines Gels oder eines Schaums vorliegt.

9. Verfahren nach einem der vorhergehenden Ansprüche, **dadurch gekennzeichnet, dass** die Temperatur bis zu einer Temperatur zwischen 60°C und 250°C, vorzugsweise zwischen 120°C und 220°C, erhöht wird.

10. Verfahren nach einem der vorhergehenden Ansprüche, **dadurch gekennzeichnet, dass** man die reduzierende Zusammensetzung auf feuchte und saubere Haarfasern aufbringt.

11. Verfahren nach einem der vorhergehenden Ansprüche, **dadurch gekennzeichnet, dass** man nach dem Aufbringen der reduzierenden Zusammensetzung die reduzierende Zusammensetzung vor der Anwendung des Eisens einwirken lässt.

12. Verfahren nach einem der vorhergehenden Ansprüche, **dadurch gekennzeichnet, dass** man die Haarfasern vor dem Temperaturerhöhungsschritt nicht spült.

13. Verfahren nach einem der vorhergehenden Ansprüche, **dadurch gekennzeichnet, dass** es einen Schritt der teilweisen Vortrocknung der Haarfasern vor dem Temperaturerhöhungsschritt umfasst.

14. Verwendung des Verfahrens nach einem der vorhergehenden Ansprüche zur dauerhaften Modifizierung der Form des Haars mit geringer Degradation der Farbe der Haare und/oder mit geringer Degradation der Haarfasern.

## Claims

1. Method for treating hair fibres, without additional application of a composition containing a chemical oxidizing agent, **characterized in that** it comprises the following steps:
- applying to the hair fibres a ceramide-free reducing composition containing at least one reducing agent and at least one cosmetic active agent, the reducing agent(s) being chosen from thiols, and the cosmetic active agent(s) being chosen from polymeric active agents chosen from linear or cyclic, volatile or non-volatile silicones and cationic, anionic or amphoteric non-silicone polymers,
- increasing the temperature of the hair fibres, by means of a heating iron, to a temperature at least equal to 60°C, the increasing of the temperature being carried out before or after optional rinsing of the hair fibres.

2. Method according to Claim 1, **characterized in that** the reducing composition does not contain dithiodiglycolic acid or a salt thereof.

3. Method according to either one of the preceding claims, **characterized in that** the polymeric cosmetic active agent(s) represent(s) from 0.1% to 30%, preferably from 0.5% to 10%, by weight relative to the total weight of the reducing composition.

4. Method according to any one of the preceding claims, **characterized in that** the thiol(s) represent(s) from 0.1% to 30%, preferably from 0.5% to 20%, better still from 1% to 10%, by weight relative to the total weight of the reducing composition.

5. Method according to Claim 4, **characterized in that** the thiol(s) represent(s) less than 5% by weight of the total weight of the reducing composition.

6. Method according to any one of the preceding claims, **characterized in that** the thiol(s) is (are) chosen from cysteine and derivatives thereof, cysteamine and derivatives thereof, thiolactic acid and esters thereof, thioglycolic acid and esters thereof, thioglycerol, or salts thereof.

7. Method according to any one of the preceding claims, **characterized in that** the reducing composition comprises one or more solvents chosen from water, C₁-C₆ alcohols, preferably alkanols such as ethanol, propanol and isopropanol, polyols such as glycerol, propylene glycol and pentanediol, benzyl alcohol, polyol ethers, C₂-C₆ esters, N-methylpyrrolidone (NMP), and C₃-C₆ ketones.

8. Method according to any one of the preceding claims, **characterized in that** the reducing composition is in the form of a thickened or non-thickened lotion, a cream, a gel or a mousse.

9. Method according to any one of the preceding claims, **characterized in that** the temperature is increased up to a temperature of between 60°C and 250°C, preferably between 120°C and 220°C.

10. Method according to any one of the preceding claims, **characterized in that** the reducing composition is applied to clean wet hair fibres.

11. Method according to any one of the preceding claims, **characterized in that**, after applying the reducing composition, said composition is left on before applying the iron.

12. Method according to any one of the preceding claims, **characterized in that** the hair fibres are not rinsed before the temperature increasing step.

13. Method according to any one of the preceding claims, **characterized in that** it comprises a step of partially predrying the hair fibres before the temperature increasing step.

14. Use of the method according to any one of the preceding claims, for modifying in a long-lasting manner the shape of the head of hair with a low hair colour degradation and/or with a low fibre degradation.
